(19) Europäisches Patentamt

European Patent Office

Office européen des brevets

(11) **EP 1 505 136 A2**

(12) **EUROPEAN PATENT APPLICATION**

(43) Date of publication:
**09.02.2005 Bulletin 2005/06**

(51) Int Cl.⁷: **C09J 7/02**

(21) Application number: **04018337.8**

(22) Date of filing: **03.08.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **08.08.2003 JP 2003290237**

(71) Applicant: **Nitto Denko Corporation
Ibaraki-shi, Osaka 567-8680 (JP)**

(72) Inventors:
• **Okada, Katsuhiro Nitto Denko Corporation
Osaka 567-8680 (JP)**
• **Sasaki, Yasuyuki Nitto Denko Corporation
Osaka 567-8680 (JP)**
• **Suzuki, Seishi Nitto Denko Corporation
Osaka 567-8680 (JP)**
• **Kuniya, Masayoshi Nitto Denko Corporation
Osaka 567-8680 (JP)**

(74) Representative: **Albrecht, Thomas, Dr. et al
Kraus & Weisert
Patent- und Rechtsanwälte
Thomas-Wimmer-Ring 15
80539 München (DE)**

(54) **Pressure-sensitive adhesive sheet and method for producing the same**

(57) To provide a pressure-sensitive adhesive sheet that is free of swelling or deformation of a substrate due to migration of a liquid component from the pressure-sensitive adhesive layer and has acceptable processability, the pressure-sensitive adhesive sheet includes a carrier film; an elastomer film provided on one surface of the carrier film; and a pressure-sensitive adhesive layer provided on the elastomer film, in which the pressure-sensitive adhesive layer includes a composition that contains an acrylic polymer as a main component and a compatible component being compatible with the acrylic polymer and liquid or paste-like at room temperature, and in which the carrier film has a heat shrinkage of 3.0% or more and 4.5% or less in the longitudinal direction and 1.2% or more and 4.2% or less in the transverse direction.

**Description**

BACKGROUND OF THE INVENTION

Field of the Invention

The present invention relates to a pressure-sensitive adhesive sheet, for example, a pressure-sensitive adhesive sheet for medical use or as a hygiene material as well as to a method for producing it.

Description of Related Art

Pressure-sensitive adhesive tapes for medical use or as a hygiene material must be able to prevent penetration of water, microorganisms, viruses and the like from outside and have flexibility sufficient to follow up a curved surface or motion of the skin. For this purpose, generally such pressure-sensitive adhesive tapes use elastomer films made of elastomers such as polyurethane having high rubber elasticity as a substrate. Further, pressure-sensitive adhesive tapes for medical use or as a hygiene material, for example, dressing or the like are required to have excellent moisture permeability so that moisture produced by perspiration from the skin can be transpired. Poor moisture permeability of a pressure-sensitive adhesive tape results in pooling of moisture produced by the skin between the skin and the pressure-sensitive adhesive layer, thus causing a decrease in adhesive strength of the pressure-sensitive adhesive layer, with the result that the fixing function of the pressure-sensitive adhesive sheet is lost or the skin is macerated due to the moisture pooled on the surface of the skin so that skin disorder tends to occur. Accordingly, elastomer films that have low stress and are very thin are used as a substrate for the dressing and the like; elastomer films that have a 50% elongation modulus of about 10 N/mm$^2$ or less and a thickness of about 70 μm or less are frequently used.

Incidentally, pressure-sensitive adhesive sheets including elastomer films that have low stress and are very thin have poor processability upon its manufacture and poor workability upon its application. Hence, to aid elastomer films, JP-A-7-16258, for example, discloses a pressure-sensitive adhesive sheet having a carrier film of a synthetic resin film, paper or the like with an elastomer film temporarily bonded and carried thereon, i.e., a pressure-sensitive adhesive sheet having a three-layer structure of pressure-sensitive adhesive layer/elastomer film/carrier film.

Pressure-sensitive adhesives used in the pressure-sensitive adhesive layer made of (meth) acrylate ester polymers having excellent adhesive properties and excellent moisture permeability and low chemical irritation to the skin are generally used. Since pressure-sensitive adhesives made of (meth) acrylate ester polymers have strong adhesive strength, pressure-sensitive adhesive tapes including such pressure-sensitive adhesives cause users to feel pain when they are peeled off from the skin or they may injure stratum corneum or epidermis of the skin. Particularly, when pressure-sensitive adhesive tapes are applied on the same site repeatedly, they may sometimes cause injury of the skin accompanying bleeding, which has been a big problem.

To decrease the physical irritation to the skin as mentioned above, JP-A-6-23029 and JP-A-6-319793, for example, disclose pressure-sensitive adhesives that include a (meth)acrylate ester polymer and a relatively large amount of liquid component compatible to the polymer and is cross-linked to form a gel. For example, such pressure-sensitive adhesives can relax and disperse stress to be given to the skin upon peeling off from the skin while retaining high adhesion of the (meth)acrylate ester polymer. Therefore, the pressure-sensitive adhesives have less physical irritation to the skin and cause no peeling of stratum corneum and the like, so that they are used in transdermal drug delivery patches and medical surgical tapes.

However, when the pressure-sensitive adhesives mentioned above are laminated on an elastomer film that is temporarily bonded and carried on the above-mentioned carrier film to form a pressure-sensitive adhesive tape, the liquid component in the pressure-sensitive adhesive migrates into the elastomer film and carrier film to swell the film, thus causing deformation of the film. As a result, processability of punching and the like of the pressure-sensitive adhesive sheet has been extremely deteriorated.

SUMMARY OF THE INVENTION

The present invention has been made under the above-mentioned circumstances and it is an object of the present invention to provide a pressure-sensitive adhesive sheet that is free of swelling or deformation of a substrate due to migration of a liquid component from the pressure-sensitive adhesive layer and has acceptable processability and storage stability.

To achieve the above-mentioned object, the pressure-sensitive adhesive sheet of the present invention includes a carrier film; an elastomer film provided on one surface of the carrier film; and a pressure-sensitive adhesive layer provided on the elastomer film, in which the pressure-sensitive adhesive layer includes a composition that contains an acrylic polymer as a main component and a compatible component being compatible with the acrylic polymer and liquid or paste-like at room temperature, and in which the carrier film has a heat shrinkage of 3.0% or more and 4.5% or less in the longitudinal direction and 1.2% or more and 4.2% or less in the transverse direction.

The method for producing a pressure-sensitive adhesive sheet according to the present invention includes: laminating a carrier film and an elastomer film; applying a pressure-sensitive adhesive layer separately prepared on a release sheet to a surface of the elastomer film; and subjecting the pressure-sensitive adhesive layer to cross-linking reaction.

The above and other objects, effects, features and advantages of the present invention will become more apparent from the detailed description to follow.

DETAILED DESCRIPTION

The pressure-sensitive adhesive sheet of the present invention includes a substrate and a pressure-sensitive adhesive layer thereon. The substrate includes an elastomer film that has a carrier film on one surface thereof. It is preferable that the elastomer film has satisfactory ability to follow up the skin and satisfactory flexibility such that it can follow up unevenness or stretching and contraction of the skin accompanying bending or the like motion of the body and so on. Examples of material of such an elastomer film include polyethylene, polyvinyl chloride, ethylene-vinyl acetate copolymer, polyamide, polyester, polyurethane, and acrylic polymer. In the present invention, the elastomer film is preferably one that does not prevent perspiration from the skin. For example, polyamide, polyester, polyurethane, or acrylic polymer from which films having excellent moisture permeability can be formed are used advantageously. In particular, polyester polyurethane, polyether polyurethane, polyether polyester, and polyether polyamide are preferably used.

The polyether polyurethane is obtainedby reaction a polyol component and a polyisocyanate component. It is preferable that the polyol component that constitutes the polyether polyurethane contains at least one polyether polyol having a polyoxyalkylene skeleton.

The polyether polyol having a polyoxyalkylene skeleton can be obtained by polymerizing at least one alkylene oxide such as ethylene oxide, propylene oxide, and butylene oxide. The polyether polyol having a polyoxyalkylene skeleton having a molecular weight of 500 to 3,000 is preferably used.

In the present invention, the polyol component may be a mixture of the polyether polyol having a polyoxyalkylene skeleton and a relatively small amount of another polyol, for example, polyoxytetramethylene glycol, or a polyol such as butanediol, heptanediol, hexanediol, or cyclohexanedimethanol.

The polyether polyurethane prepared from the polyol component containing a polyether polyol having a polyoxyalkylene skeleton has a low absorbability for the pressure-sensitive adhesive prepared from a carboxylic acid ester as described below. Although the reason for this is not clear enough, it is presumed that the polyether structure having high polarity that exists in the polyether polyurethane will cause the polyether polyurethane to have decreased affinity for carboxylic acid esters having high hydrophobicity, such as, for example, carboxylic acid esters having 16 or more carbon atoms.

The polyisiocyanate component that constitute the above-mentioned polyether polyurethane may be a conventional material. For example, aromatic, aliphatic, and alicyclic diisocyanates, dimers, trimers and so on of the diisocyanates maybeused. Examples of the aromatic, aliphatic, and alicyclic diisocyanates include tolylene diisocyanate, diphenylmethane diisocyanate, hexamethylene diisocyanate, xylylene diisocyanate, hydrogenated xylylene diisocyanate, isophorone diisocyanate, hydrogenated diphenylmethane diisocyanate, 1,5-naphthylene diisocyanate, 1,3-phenylene diisocyanate, 1,4-phenylene diisocyanate, butane-1,4-diisocyanate, 2,2,4-trimethylhexamethylene diisocyanate, 2,4,4-trimethylhexamethylene diisocyanate, cyclohexane-1,4-diisocyanate, dicyclohexylmethane-4,4-diisocyanate, 1,3-bis(isocyanatomethyl)cyclohexane, methylcyclohexane diisocyanate, and m-tetramethylxylylene diisocyanate. Also, dimers and trimers of these and polyphenylmethane polyisocyanates may be used. The trimers include isocyanurate type, biuret type, allophanate type and so on and are used as appropriate. The polyisocyanates may be used singly or as combinations of two or more of them.

In the present invention, further a chain extender may be used. The chain extender may be a conventional material. Examples of the chain extender that can be used include diols such as ethylene glycol, propylene glycol, and butanediol; diamines such as ethylenediamine, tolylenediamine, isophoronediamine.

In the present invention, usually, additives that are commonly used in films, for example, ultraviolet absorbents, antioxidants, fillers, pigments, colorants, flame retardants, and antistatic agents may be added as necessary. These additives are used in ordinary amounts depending on the kind.

The polyether polyurethane can be polymerized by using a one-shot method or a prepolymer method. Further, a bulk polymerization method that does not employ a solvent may be used. To reduce viscosity, polymerization may be performed in a solution.

Hereinafter, the bulk polymerization will be explained concretely. Polyol is charged in a reactor and while stirring to adjust the temperature at 50 to 80°C, polyisocyanate is added to cause urethanation. Further, a chain extender is added and allowed to react, the reaction product is transferred into a tray and held at 100 to 150°C for 4 hours or more to complete the reaction. Thus, bulky polyether polyurethane can be obtained.

A film made of polyether polyurethane can be formed by pulverizing bulky polyether polyurethane to give resin pellets, melting the resin pellets, and then extruding the molten resin into a film in the form of a sheet using a T die extruder or inflation die extruder. The film extruded in the form of a sheet is usually wound up. Alternatively, the extruded film may be rolled and extended through two hot rolls to form a film consisting of polyether polyurethane and the film is wound up as necessary. Further, a polyether polyurethane film may be formed by dissolving resin pellets in a solvent such as N,N-dimethylamide (DMF), coating the resulting solution on a release liner such as a polyester film by using a bar coater or the like, and drying the coating to remove the solvent.

In the present invention, the thickness of the elastomer film is preferably in the range of 10 to 150 μm, more preferably 20 to 70 μm in the case of a pressure-sensitive adhesive sheet for medical use or as a hygiene material. If the film thickness is less than 10 μm, the film tends to be broken when it is applied to and peeled from the skin, so that handleability of the film is deteriorated. On the other hand, if the film thickness is greater than 150 μm, moisture permeability becomes insufficient or follow-up property of the film to the skin may become poor. When the pressure-sensitive adhesive sheet is used for dressing application, the thickness of the elastomer film is preferably in the range of 20 to 60 μm.

In the present invention, an elastomer film onto which a carrier film is laminated is used as a substrate. Lamination of the carrier film can improve processability of the film upon manufacture and handleability of the film upon application.

It is preferable that the carrier film and elastomer film are transparent or translucent so that when the pressure-sensitive adhesive sheet is applied to the skin, the application site on the skin can be visually confirmed through the pressure-sensitive adhesive sheet. Further, the carrier film is preferably one that can impart high elastic modulus to the elastomer film. For example, the carrier film has preferably a 50% elongation modulus by a tensile test at normal temperature and normal humidity of 2 N/mm$^2$ or more and 200 N/mm$^2$ or less, more preferably 8 N/mm$^2$ or more and 50 N/mm$^2$ or less.

The carrier film to be used must have a heat shrinkage of 3.0% or more and 4.5% or less in the longitudinal direction and 1.2% or more and 4.2% or less in the transverse direction. As will be described hereinafter, the pressure-sensitive adhesive used in the pressure-sensitive adhesive layer is preferably a pressure-sensitive adhesive that contains a specified carboxylic acid ester. When such a pressure-sensitive adhesive is used, it is preferable that heat crosslinking treatment is performed in order to increase the pressure-sensitive adhesiveness. In the case where such heat crosslinking treatment is performed, it is preferable that the carrier film is drawn before the treatment; it is necessary that the carrier film has a predetermined heat shrinkage. If the heat shrinkage of the carrier film is less than 3.0% in the longitudinal direction and less than 1.2% in the transverse direction, the liquid component in the pressure-sensitive adhesive migrates into the carrier film through the elastomer film, thus causing swelling and deformation of the substrate. Further, if the heat shrinkage is higher than 4.5% in the longitudinal direction and higher than 4.2% in the transverse direction, the heat shrinkage of the carrier film is higher than is necessary, thus decreasing productivity of the pressure-sensitive adhesive sheet.

The heat shrinkage of the carrier film is measured as follows. That is, a carrier film to be measured is cut into a square of a size of 100 mm × 100 mm and a length (L1) of longitudinal (or transverse) side of the square is measured by using a vernier caliper. Then, the cut out base material (square carrier film) is heated at 130°C for 15 minutes and a length (L2) of longitudinal (or transverse) side of the square after heating is measured. The heat shrinkage is obtained by the following equation.

$$\text{Heat shrinkage (\%)} = [(L1 - L2)/L1] \times 100$$

The materials of the carrier film that can be used include polyolefins such as polyethylene and polypropylene; polyesters such as polyethylene terephthalate; polyamides such as nylon; polyvinyl chloride, polyvinylidene chloride and so on.

The pressure-sensitive adhesive sheet of the present invention has a pressure-sensitive adhesive layer on the substrate. More specifically, the pressure-sensitive adhesive layer has a pressure-sensitive adhesive layer on the above-mentioned elastomer film of the substrate. The pressure-sensitive adhesive layer can be obtained by mixing an acrylic polymer, a carboxylic acid ester compatible to the acrylic polymer, and a crosslinking agent, and subjecting the mixture to crosslinking.

Here, the acrylic polymer refers to a polymer of (meth)acrylic acid ester as a main component and a monomer copolymerizable therewith as necessary. The (meth)acrylic acid esters are those that have two or more carbon atoms, preferably 2 or more and 18 or less carbon atoms in the alkyl group. Examples of the (meth) acrylic acid ester include ethyl, propyl, butyl, pentyl, hexyl, octyl, nonyl, decyl, dodecyl, etc. esters of (meth) acrylic acid. In the present invention, one or more of these (meth) acrylic acid esters are used. The alkyl ester chain may be either straight chain or branched chain.

The monomers copolymerizable with (meth)acrylic acid esters include, for example, carboxylic group-containing monomers such as (meth) acrylic acid, itaconic acid, and maleic acid; hydroxyl group-containing monomers such as 2-hydroxylethyl (meth)acrylate and 2-hydroxypropyl (meth)acrylate; alkoxy group-containing monomers such as methoxyethyl (meth)acrylic acid, ethoxyethyl (meth)acrylic acid, butoxyethyl (meth)acrylic acid, ethylene glycol (meth) acrylic acid, methoxypolyethylene glycol (meth) acrylic acid, and ethoxydiethylene glycol (meth)acrylic acid; vinyl group-containing monomers such as styrene and styrene derivatives, vinyl acetate, N-vinyl-2-pyrrolidone; and the like. One or more of these monomers may be copolymerized with (meth)acrylic acid esters as necessary.

It is desirable that the acrylic polymer has a glass transition temperature of 260°K or less. Use of the acrylic polymer having a glass transition temperature of 260°K or less allows the pressure-sensitive adhesive to exhibit sufficient adhesion to the skin, so that it can be used advantageously as a pressure-sensitive adhesive layer in a pressure-sensitive adhesive sheet for medical use or as a hygiene material.

The acrylic polymer can be obtained by a conventional polymerization method such as, for example, a solution polymerization method, an emulsion polymerization method, and a suspension polymerization method. The acrylic polymer can be obtained by performing radical polymerization with a radical polymerization initiator such as a peroxide compound or an azo compound.

The carboxylic acid ester to be mixed with the acrylic polymer preferably has 16 or more carbon atoms and is preferably liquid or paste-like at normal temperature. It should be noted that examples of the carboxylic acid ester that can be used do not include those carboxylic acid esters that are solid, such as wax-like ones. This is because if a solid carboxylic acid ester is mixed in forming a pressure-sensitive adhesive layer, the resulting pressure-sensitive adhesive layer has a decreased adhesion.

In the present invention, mixing an acrylic polymer, a carboxylic acid ester and a crosslinking agent and forming crosslinking at least partly can give rise to a gel-like pressure-sensitive adhesive layer. The pressure-sensitive adhesive layer thus obtained can have a decreased elastic modulus in a minute deformation region, resulting in an improvement in adherence (wetting) of the surface of the pressure-sensitive adhesive layer to the uneven surface of the skin, thus providing good adhesion to the skin. In addition, when the pressure-sensitive adhesive sheet is peeled off from the skin, the stress applied to the surface of skin can be alleviated or dispersed, so that the pressure-sensitive adhesive layer has the following effects. Upon the peeling off, substantially no physical irritation is given to the surface of skin. Substantially no stratum corneum comes off from the surface of skin. Further, damage of the skin is minimized.

Examples of the carboxylic acid ester that can be used preferably include esters of monohydric alcohols such as ethyl myristate, isopropyl myristate, isopropyl palmitate, butyl stearate, isopropyl isostearate, hexyl laurate, cetyl lactate, myristyl lactate, diethyl phthalate, dioctyl phthalate, octyl dodecyl myristate, octyl dodecyl oleate, hexyl decyl dimethyloctanoate, cetyl 2-ethylhexanoate, isocetyl 2-ethylhexanoate, stearyl 2-ethylhexanoate, and dioctyl succinate, and esters of dihydric or higher polyhydric alcohols, such as propylene glycol dicaprylate, propylene glycol dicaprate, propylene glycol diisostearate, glyceryl monocaprylate, glyceryl tricaprylate, glyceryl tri-2-ethylhexanoate, glyceryl tricaprate, glyceryl trilaurate, glyceryl triisostearate, glyceryl trioleate, and trimethylolpropane tri-2-ethylhexanoate.

In the present invention, it is preferable that at least one kind of the above-mentioned carboxylic acid esters is dissolved in the acrylic polymer. The amount of the carboxylic acid ester to be blended is not particularly limited. For example, preferably 30 to 100 mass parts of the carboxylic acid ester is contained in 100 mass parts of the acrylic polymer.

In the present invention, to improve the pressure-sensitive adhesiveness, it is preferable that a part of the acrylic polymer having dissolved therein the above-mentioned carboxylic acid ester is crosslinked. To form crosslinking, crosslinking treatment is performed. For example, chemical crosslinking treatments using organic peroxides, isocyanate compounds, organomet salts, metal chelate compounds, epoxy group-containing compounds and the like may be performed. Alternatively, physical crosslinking treatments using ionized radiations.

The resin composition that forms a pressure-sensitive adhesive layer may be blended with plasticizers such as glycerin and polyethylene glycol; water-soluble or water-absorbing resins such as polyacrylic acid and polyvinylpyrrolidone; tackifiers such as rosin-, terpene-, and petroleum-based ones; various additives such as various softeners, fillers, and pigments, as necessary. In particular, when a carboxylic acid ester having an unsaturated bond is used as the carboxylic acid ester, it is feared that changes in physical properties as a result oxidation deterioration due to the oxygen in the atmosphere, so that desired characteristics of the pressure-sensitive adhesive are not obtained, so that it is preferable that a conventional antioxidant is blended into the resin composition.

It is preferable that the thickness of the pressure-sensitive adhesive layer is set within the range of 10 to 80 μm. If the thickness of the pressure-sensitive adhesive layer is less than 10 μm, no sufficient adhesion can in some cases be obtained while the pressure-sensitive adhesive sheet is applied to the skin. On the other hand, if the thickness of the pressure-sensitive adhesive layer is greater than 80 μm, moisture permeability of the level that is required for the pressure-sensitive adhesive sheet for application to the skin cannot be obtained in some cases.

In the present invention, medical tapes and sheets such as adhesive plasters can be formed by using the above-mentioned the pressure-sensitive adhesive sheet. For example, the pressure-sensitive adhesive sheet can be cut to a suitable size to form an adhesive plaster, a dressing for covering a wound portion, a protector to be used after surgical operation, housing for a needle of a catheter, or a medical tape or sheet such as a covering material for gauze. Further, the pressure-sensitive adhesive sheet may be combined with other base material to form medical products such as a tape for fixing and a tape for holding an instrument.

It should be noted that the term "film" as used herein includes sheet and the term "sheet" as used herein includes film.

According to the present invention, pressure-sensitive adhesive sheets that have appropriate adhesive strength, can suppress physical irritation to be applied to an adherend when they are peeled off therefrom, and do not cause skin disorder can be provided. Further, according to the present invention, pressure-sensitive adhesive sheets that do not cause swelling deformation of the substrate due to migration of the liquid component from the pressure-sensitive adhesive layer and hence have acceptable processability and storage stability can be provided.

Examples

Hereinafter, the present invention will be described in more detail by way of examples. However, the present invention should not be considered as being limited thereto and various modifications can be made without departing the scope and spirit of the present invention. It should be noted that in the following examples, "part" or "parts" means "mass part" or "mass parts".

Example 1

Polyether polyurethane (manufactured by Dainichiseika Color & Chemicals Mfg. Co., Ltd., "RESAMIN P-210") as an elastomer resin was heat melted in a twin-screw type extruder and then extruded from a T-die extruder to a thickness of 30 μm to form an elastomer film.

As a carrier film, an elongated polypropylene film (manufactured by Gunze Ltd., "SILFAN MTO", thickness 40 μm) having a heat shrinkage of 4.5% in the longitudinal direction and 4.2% in the transverse direction was provided and the formed elastomer film was adhered onto the elongated polypropylene film by using a rubber roll to obtain a substrate.

Then, 60 parts of glyceryl tricaprylate that was liquid or paste-like at normal temperature and 0.075 part of trifunctional isocyanate compound as a crosslinking agent component and 100 parts (solids weight) of a solvent-type acrylic pressure-sensitive adhesive containing as a main component a copolymer of isononyl acrylate, 2-methoxyethyl acrylate, and acrylic acid in a weight ratio of 65: 30: 5 were mixed and dissolved in toluene to form a pressure-sensitive adhesive solution. The obtained pressure-sensitive adhesive solution was coated on a release-treated surface of a release sheet to a dry thickness of 30 μm and dried at 120°C for 3 minutes to form a pressure-sensitive adhesive layer. Then, the surface of pressure-sensitive adhesive layer was superposed on a surface of the elastomer film of the formed substrate and bonded together by using a rubber roll. Thereafter, the resultant was stored in an atmosphere of 60°C for 3 days to allow the crosslinking reaction of the pressure-sensitive adhesive layer to be completed, thus preparing a pressure-sensitive adhesive sheet.

Example 2

A pressure-sensitive adhesive sheet was prepared in the same manner as in Example 1 except that in Example 1, the carrier film was replaced by an elongated polypropylene film (manufactured by Gunze Ltd., "SILFAN MTO", thickness 40 μm) having a heat shrinkage of 3.0% in the longitudinal direction and 1.2% in the transverse direction.

Example 3

A pressure-sensitive adhesive sheet was prepared in the same manner as in Example 1 except that in Example 1, the carrier film was replaced by an elongated polypropylene film (manufactured by Gunze Ltd., "SILFAN MTO", thickness 40 μm) having a heat shrinkage of 3.4% in the longitudinal direction and 1.3% in the transverse direction.

Comparative Example 1

A pressure-sensitive adhesive sheet was prepared in the same manner as in Example 1 except that in Example 1, the carrier film was replaced by an elongated polypropylene film (manufactured by Gunze Ltd., "SILFAN MTO",

thickness 40 μm) having a heat shrinkage of 2.6% in the longitudinal direction and 0.3% in the transverse direction.

Comparative Example 2

A pressure-sensitive adhesive sheet was prepared in the same manner as in Example 1 except that in Example 1, the carrier film was replaced by an elongated polypropylene film (manufactured by Gunze Ltd., "SILFAN MTO", thickness 40 μm) having a heat shrinkage of 2.6% in the longitudinal direction and 0.5% in the transverse direction.

Comparative Example 3

A pressure-sensitive adhesive sheet was prepared in the same manner as in Example 1 except that in Example 1, the carrier film was replaced by an elongated polypropylene film (manufactured by Gunze Ltd., "SILFAN MTO", thickness 40 μm) having a heat shrinkage of 2.8% in the longitudinal direction and 0.5% in the transverse direction.

Comparative Example 4

A pressure-sensitive adhesive sheet was prepared in the same manner as in Example 1 except that in Example 1, the carrier film was replaced by an elongated polypropylene film (manufactured by Gunze Ltd., "SILFAN MTO", thickness 40 μm) having a heat shrinkage of 3.0% in the longitudinal direction and 0.7% in the transverse direction.

Comparative Example 5

A pressure-sensitive adhesive sheet was prepared in the same manner as in Example 1 except that in Example 1, the carrier film was replaced by an elongated polypropylene film (manufactured by Gunze Ltd., "SILFAN MTO", thickness 40 μm) having a heat shrinkage of 2.6% in the longitudinal direction and 0.5% in the transverse direction and the crosslinking treatment of the pressure-sensitive adhesive layer was performed by storing it in an atmosphere at 23°C for 3 days to allow the crosslinking reaction of the pressure-sensitive adhesive layer to be completed.

Comparative Example 6

A pressure-sensitive adhesive sheet was prepared in the same manner as in Example 1 except that in Example 1, the carrier film was replaced by an elongated polypropylene film (manufactured by Gunze Ltd., "SILFAN MTO", thickness 40 μm) having a heat shrinkage of 2.6% in the longitudinal direction and 0.5% in the transverse direction and the crosslinking treatment of the pressure-sensitive adhesive layer was performed by storing it in an atmosphere at 40°C for 3 days to allow the crosslinking reaction of the pressure-sensitive adhesive layer to be completed.

<<Evaluation Tests>>

The pressure-sensitive adhesive sheets obtained in Examples 1 to 3 and Comparative Examples 1 to 6 were subjected to evaluation tests as described below. Table 1 shows the results.

(1) Deformation ratio

Test pieces of a size of 100 mm × 100 mm were cut out from the pressure-sensitive adhesive sheet before the crosslinking reaction and the length (L1) of the longitudinal (or transverse) side of each test piece was measured by using a vernier caliper. Then, the test pieces were subjected to the crosslinking reactions shown in Examples 1 to 3 and Comparative Examples 1 to 6, respectively, and the length (L2) of the longitudinal (or transverse) side of each of the test pieces after the crosslinking reaction was measured. The measured values of L1 and L2 were assigned in the following equation to obtain a deformation ratio.

$$\text{Deformation ratio (\%)} = \{(L1 - L2)/L1\} \times 100$$

(2) Amount of migration

The release sheet was peeled off from each of the pressure-sensitive adhesive sheets and the surface of each pressure-sensitive adhesive layer was applied to an aluminum plate and in this state, the carrier film was peeled off from the elastomer film. A test piece of a size of 30 mm × 40 mm was cut out from each carrier film thus peeled.

Each of the obtained test pieces was immersed in 50 cc of ethyl acetate at normal temperature for 24 hours to effect extraction. The extract solution after the immersion was injected into a capillary gas chromatography analyzer and the amount of the liquid component that migrated into the carrier film was quantated from a peak area of a chromatogram.

(3) Productivity

The pressure-sensitive adhesive sheets after the crosslinking treatment were observed with naked eye on the state of lifting up of the pressure-sensitive adhesive layer from the release sheet and the state of deformation of the pressure-sensitive adhesive sheet. Evaluation was made based on the following standards.

Standards of evaluation

"○": Neither lifting up of the pressure-sensitive adhesive layer from the release sheet nor deformation of the pressure-sensitive adhesive sheet was observed at all.
" X " : Either lifting up of the pressure-sensitive adhesive layer from the release sheet or deformation of the pressure-sensitive adhesive sheet was observed.

(4) Storage stability

After being stored in incubator at 50°C for 1 month, the pressure-sensitive adhesive sheets were observed with naked eye for the state of lifting up of the pressure-sensitive adhesive layer from the release sheet, the state of deformation of the pressure-sensitive adhesive sheet, and curl of the pressure-sensitive adhesive sheet, and evaluation was made based on the following standards.

Standards of evaluation

"○": Neither lifting up of the pressure-sensitive adhesive layer from the release sheet nor deformation of the pressure-sensitive adhesive sheet was observed at all. Further, no curl occurred.
"X" : Either lifting up of the pressure-sensitive adhesive layer from the release sheet or deformation of the pressure-sensitive adhesive sheet was observed, or curl occurred in the pressure-sensitive adhesive sheet.

Table 1

| | Heat shrinkage (%) | | Cross-linking reaction | Deformation ratio (%) | | Amount of Migration (g/m$^2$) | Productivity | Storage Stability |
|---|---|---|---|---|---|---|---|---|
| | Longitudinal | Transverse | | Longitudinal | Transverse | | | |
| Ex. 1 | 4.5 | 4.2 | 60°C× 3 Days | 0.5 | 0.2 | 1.1 | ○ | ○ |
| Ex. 2 | 3.0 | 1.2 | 60°C× 3 Days | 1.0 | 0.9 | 1.2 | ○ | ○ |
| Ex. 3 | 3.4 | 1.3 | 60°C× 3 Days | 1.0 | 0.7 | 1.1 | ○ | ○ |
| Comp. Ex. 1 | 2.6 | 0.3 | 60°C× 3 Days | 1.6 | 1.2 | 1.3 | × | × |
| Comp. Ex. 2 | 2.6 | 0.5 | 60°C× 3 Days | 1.2 | 1.1 | 1.3 | × | × |
| Comp. Ex. 3 | 2.8 | 0.5 | 60°C× 3 Days | 1.1 | 1.1 | 1.3 | × | × |
| Comp. Ex. 4 | 3.0 | 0.7 | 60°C× 3 Days | 1.1 | 1.0 | 1.2 | × | × |
| Comp. Ex. 5 | 2.6 | 0.5 | 23°C× 3 Days | 0.1 | 0.1 | 0.1 | ○ | × |
| Comp. Ex. 6 | 2.6 | 0.5 | 40°C× 3 Days | 0.5 | 0.4 | 0.6 | ○ | × |

The results in Table 1 indicate that thepressure-sensitive adhesive sheets of Examples 1 to 3 of the present invention showed low deformation ratios and excellent productivity and storage stability. Further, they show small amounts of migration of the liquid component in the pressure-sensitive adhesive.

On the other hand, the pressure-sensitive adhesive sheets of Comparative Examples 1 to 4 showed high deformation ratios and poor productivity. The pressure-sensitive adhesive sheets of Comparative Examples 5 and 6 showed small amounts of migration of the liquid component in the pressure-sensitive adhesive and low deformation ratios but they showed migration of the liquid component in the pressure-sensitive adhesive during the storage. They also showed lifting up of the pressure-sensitive adhesive layer from the release sheet, deformation of the pressure-sensitive adhesive sheet, and curl of the pressure-sensitive adhesive sheet. Therefore, the pressure sensitive adhesive sheets of these comparative examples were practically applicable.

According to the present invention, preparation of a pressure-sensitive adhesive sheet by using a substrate having a specified carrier film enables to suppress swelling deformation of the substrate to a very low level even if the liquid component contained in the pressure-sensitive adhesive layer migrates into the substrate during the manufacture of the pressure-sensitive adhesive sheet, so that pressure-sensitive adhesive sheet having excellent productivity and excellent storage stability can be obtained.

Further, the pressure-sensitive adhesive sheet of the present invention has flexibility to follow up the curve of the skin and motion of the body, so that it does not undergo peeling and the like when applied to a finger or the like as an adhesive plaster, for example, and the finger performs curvature movement.

The present invention may be embodied in other specific forms without departing from the spirit or essential characteristics thereof. The present embodiment is therefore to be considered in all respects as illustrative and not restrictive, the scope of the invention being indicated by the appended claims rather than by the foregoing description and all changes which come within the meaning and range of equivalency of the claims are therefore intended to be embraced therein.

## Claims

1.  A pressure-sensitive adhesive sheet comprising:

    a carrier film;
    an elastomer film provided on one surface of the carrier film; and
    a pressure-sensitive adhesive layer provided on the elastomer film, wherein the pressure-sensitive adhesive layer includes a composition that contains an acrylic polymer as a main component and a compatible component being compatible with the acrylic polymer and liquid or pas te-like at room temperature,

    wherein the carrier film has a heat shrinkage of 3.0% or more and 4.5% or less in the longitudinal direction and 1.2% or more and 4.2% or less in the transverse direction.

2.  The pressure-sensitive adhesive sheet according to claim 1, wherein the elastomer film comprises one selected from the group consisting of polyester polyurethane, polyether polyurethane, polyether polyester, and polyether polyamide.

3.  The pressure-sensitive adhesive sheet according to claim 1, wherein the carrier film comprises one selected from the group consisting of polyethylene, polypropylene, and polyester.

4.  The pressure-sensitive adhesive sheet according to claim 1, wherein the carrier film has a 50% elongation modulus at normal temperature and normal humidity of 2 $N/mm^2$ or more and 200 $N/mm^2$ or less.

5.  The pressure-sensitive adhesive sheet according to claim 1, wherein the compatible component is a carboxylic acid ester.

6.  The pressure-sensitive adhesive sheet according to claim 5, wherein the carboxylic acid ester has 16 carbon atoms or more.

7.  The pressure-sensitive adhesive sheet according to claim 1, wherein the pressure-sensitive adhesive sheet comprises 30 mass parts or more and 100 mass parts or less of the compatible component per 100 mass parts of the acrylic polymer.

8. The pressure-sensitive adhesive sheet according to claim 1, wherein the acrylic polymer has a glass transition temperature of 260°K or less.

9. The pressure-sensitive adhesive sheet according to claim 2, wherein the elastomer film comprises the polyether polyurethane, and wherein the polyether polyurethane includes a polyol component that includes at least one polyether polyol having a polyoxyalkylene skeleton.

10. The pressure-sensitive adhesive sheet according to claim 2, wherein the carrier film comprises at least one selected from the group consisting of polyethylene, polypropylene, and polyester.

11. The pressure-sensitive adhesive sheet according to claim 2, wherein the carrier film has a 50% elongation modulus at normal temperature and normal humidity of 2 N/mm$^2$ or more and 200 N/mm$^2$ or less.

12. The pressure-sensitive adhesive sheet according to claim 2, wherein the compatible component is a carboxylic acid ester.

13. The pressure-sensitive adhesive sheet according to claim 12, wherein the carboxylic acid ester has 16 carbon atoms or more.

14. The pressure-sensitive adhesive sheet according to claim 2, wherein the pressure-sensitive adhesive sheet comprises 30 mass parts or more and 100 mass parts or less of the compatible component per 100 mass parts of the acrylic polymer.

15. The pressure-sensitive adhesive sheet according to claim 2 , wherein the acrylicpolymer has a glass transition temperature of 260°K or less.

16. A method for producing a pressure-sensitive adhesive sheet, comprising:

    laminating a carrier film and an elastomer film;
    applying a pressure-sensitive adhesive layer separately prepared on a release sheet to a surface of the elastomer film; and
    subjecting the pressure-sensitive adhesive layer to cross-linking reaction.

17. The method for producing a pressure-sensitive adhesive sheet according to claim 16, wherein the carrier film has a heat shrinkage of 3.0% or more and 4.5% or less in the longitudinal direction and 1.2% or more and 4.2% or less in the transverse direction.

18. The method for producing a pressure-sensitive adhesive sheet according to claim 17, wherein the elastomer film comprises one selected from the group consisting of polyester polyurethane, polyether polyurethane, polyether polyester, and polyether polyamide.